(19) Europäisches Patentamt / European Patent Office / Office européen des brevets

(11) **EP 4 406 543 A1**

(12) **EUROPEAN PATENT APPLICATION**

(43) Date of publication:
**31.07.2024 Bulletin 2024/31**

(21) Application number: **23153122.9**

(22) Date of filing: **24.01.2023**

(51) International Patent Classification (IPC):
**A61K 33/24** (2019.01)     **A61P 31/00** (2006.01)
**A61P 31/04** (2006.01)

(52) Cooperative Patent Classification (CPC):
(C-Sets available)
**A61K 33/24; A61K 31/728; A61P 31/00;
A61P 31/04**                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **AuroDerm ApS
8240 Risskov (DK)**

(72) Inventors:
• **Bjerring, Peter
  8240 Risskov (DK)**
• **Danscher, Gorm
  8000 Århus (DK)**

(74) Representative: **AWA Denmark A/S
Strandgade 56
1401 Copenhagen K (DK)**

(54) **GOLD PARTICLE FOR USE IN THERAPY TO PREVENT OR REDUCE THE FORMATION OF BIOFILM IN PATIENTS WHO ARE TO RECEIVE, ARE RECEIVING, OR HAVE RECEIVED SOFT TISSUE FILLER**

(57)     A gold particle having a length in the largest dimension in the range of 20 - 1000 μm for use in therapy to prevent or reduce the formation of biofilm in patients who are to receive, are receiving, or have received soft tissue filler. Composition comprising one or more gold particle(s) having a length in the largest dimension in the range of 20 - 1000 μm and a physiologically acceptable carrier for use in therapy to prevent or reduce the formation of biofilm in patients who are to receive, are receiving, or have received soft tissue filler. A medical device operable to prevent or reduce the formation of biofilm in patients who are to receive, are receiving, or have received soft tissue filler, the medical device comprising: a composition comprising one ore more gold particle(s) having a length in the largest dimension in the range 20 - 1000 μm and a physiologically acceptable carrier. A method of prevention or reduction of the formation of biofilm in a patient who is to receive, is receiving, or have received soft tissue filler, the method comprising the step of: administering a gold particle having a length in the largest dimension in the range of 20 - 1000 μm to the dermis and/or submucosal layer of the patient.

Figure 1A

EP 4 406 543 A1

(52) Cooperative Patent Classification (CPC): (Cont.)

C-Sets
**A61K 31/728, A61K 2300/00;**
**A61K 33/24, A61K 2300/00**

**Description**

**Technical Field**

The present invention relates generally to a gold particle for use in therapy to prevent or reduce the formation of biofilm in patients who are to receive, are receiving, or have received soft tissue filler.

**Background**

Microorganisms occur in several different phenotypes, including planktonic, dispersal, and aggregates. Most known is probably the planktonic phenotype, where microorganisms exist as single, independent cells. Nevertheless, the most common phenotype is when microorganisms organize into aggregates. Infections with planktonic microorganisms are generally curable with antibiotics, while infections with aggregated microorganism can be untreatable. The aggregated microorganisms form and embed themselves into a biofilm, which is a matrix of extracellular polymeric substances (EPSs). By embedding themselves into a biofilm, the aggregated microorganisms can evade the immune response. The biofilm may include polysaccharides, proteins, glycoproteins, polypeptides, lipids, DNA, and RNA. The specific composition of the biofilm depends on the species, the environment in which it is formed, and the nutrients available. Biofilms are formed by microorganisms from several different kingdoms, including bacteria, fungi, archaea, and protists. However, bacterial infections are the main cause of biofilm formation in humans. Formation of biofilm may occur on living as well as non-living surfaces and are commonly found in natural, industrial, and hospital settings. Examples of natural settings include on the surface of skin, within body cavities and on implanted devices. Other examples are, but are not limited to, bone, dental, and breast implants, catheters, and other biomedical devices suitable for implantation or insertion.

1 to 3% of patients who receive soft tissue fillers experience adverse events of which almost all arise from bacterial infections where the bacteria produce biofilm. However, it is generally agreed that this number is highly under-reported. Some of the adverse events can be serious and include vascular occlusion leading to skin necrosis or blindness, inflammatory events, and nodule formation. It is hypothesized that during injections of soft tissue filler, bacteria on the surface of the skin are guided into the soft tissue via the needle used for injection. Early infectious complications generally present as a localized skin infection, cellulitis, or abscess. Fillers can also become starting point for chronic infection. A late complication is the development of foreign body granulomas. Both gram-positive and gram-negative bacteria can form biofilms in filler related infections. Some of the most common forms are Enterococcus faecalis, Staphylococcus aureus, Staphylococcus epidermidis, Streptococcus viridans, E. coli, Klebsiella pneumoniae, Proteus mirabilis, and Pseudomonas aeruginosa.

Injection of soft tissue filler can create discontinuities that serve as a protected, microenvironmental niche for bacteria. The porous and heterogeneous solid-liquid structure of soft tissue fillers can serve as an ecological niche for bacteria while hindering the response of larger immune cells. Without the wish to be be bound by theory, it is thought that these properties of soft tissue fillers are important in the underlying mechanism of biofilm formation in soft tissue fillers and contribute to the high prevalence of biofilm in patients who have received such fillers. In addition, it is believed that the porous and heterogeneous solid-liquid structure of soft tissue fillers is what makes biofilm formation in soft tissue filler difficult to reduce or prevent, since the biofilm together with the filler form an exceptional microenvironment protecting the bacteria from pharmaceutical intervention. As such, interventions that are effective in other types of implants may not necessarily be effective in the prevention or reduction of biofilm in soft tissue filler.

In addition, procedures involving multiple needle passes are regularly used when injecting soft tissue fillers, and studies have shown that multiple needle passes significantly increase the risk of filler contamination. Without the wish to be be bound by theory, it is thought that the use of multiple needle passes further contributes to the high prevalence of biofilm in patients who have received soft tissue fillers.

The 2014 and 2015 European Society of Clinical Microbiology and Infectious Diseases (ESCMID) guidelines for the diagnosis and treatment of biofilm details consensus methods of how to diagnose biofilm in soft tissue filler infections. The guidelines mention that there is good evidence for diagnostic approaches for diagnosing biofilm formed in tissue injected with soft tissue filler. Under prevention and treatment of biofilm infections, the guidelines state that there is no treatment regimen that can be recommended regarding soft tissue fillers.

Current treatments to prevent biofilm formation include prophylactic antibiotic intervention, administrated two hours before injection of the filler, or 7 to 14 days after.

WO2017156418 A1 discloses treatment compositions for the treatment of dermal tissue, including treatment compositions useful as soft tissue fillers and/or tissue glues, which incorporate one or more cationic steroidal antimicrobial (CSA) compounds to provide anti-microbial activity.

However, antibiotic intervention is often not effective for the prevention of biofilm formation, as the efficacy heavily depends on the biofilm's composition.

In general, the great variation in biofilm composition makes it difficult to find universal therapies to prevent or reduce biofilm formation. Hence, there is a need to find a general therapy for preventing or reducing biofilm formation in patients who are to receive, are receiving, or have received soft tissue filler.

**Summary**

On this background, the present disclosure seeks to provide a gold particle for use in therapy to prevent or reduce the formation of biofilm in patients who are to receive, are receiving, or have received soft tissue filler a composition comprising one or more gold particles, a medical device for administration of a composition comprising a gold particle, and a method of treatment by administration of a gold particle.

Surprisingly, the applicant has found that administering a soft tissue filler containing, together with or prior to a gold particle according to the invention is effective in the prevention and/or reduction of biofilm formation in the tissue. At the same time, the particle sized gold causes a reduction in the size of collagen fibers i.e., the tissue remains soft.

Hence, in a first aspect the present invention relates to a gold particle having a length in the largest dimension in the range of 20 - 1000 $\mu$m for use in therapy to prevent or reduce the formation of biofilm in patients who are to receive, are receiving, or have received soft tissue filler.

Upon administration or injection of gold particles with a length in the largest dimension within the range of 20 - 1000 $\mu$m into a soft tissue containing a filler, local macrophages attach themselves to the metallic gold surfaces and release biocidal and anti-inflammatory gold ions.

The macrophages produce an ultra-thin layer, denoted a dissolution membrane, within which the macrophages control the chemical milieu including releasing cyanide ions. In this way, the macrophages slowly dissolve the outermost surface of the gold implants. The process is well-known and is called dissolucytosis, whereby gold ions, bound in gold cyanide molecule, are released into the dissolution membrane. Without the wish to be bound by any theory it is believed that it is the macrophage-liberated gold ions, liberated from pure gold according to the invention, that result in the effect of preventing or reducing the formation of biofilm that often occur in patients who have received soft tissue filler.

In the context of the present disclosure, gold should be understood as pure gold which in this context is at least 95.00% w/w, such as at least 99.00% w/w, such as at least 99.9% w/w gold, such as at least 99.99% w/w gold.

The use of pure gold is important from a safety point of view as some of the amalgamated metals in alloys can be toxic to the tissue.

Macrophage-liberated gold ions, liberated from pure gold according to the invention, do not spread further away than about 500 microns from the locality from where the metallic gold was administered. This is important from a safety point of view and reflects the fact that the amount of gold ions released is very low excluding a general exposure to the organism, i.e., the bio-released gold ions stay local.

In the context of the present disclosure dissolucytosis is the term for an extracellular liberation of gold ions, from the surface of the gold particles. The dissolucytosis takes place within the dissolution membrane and is most likely caused by the capability of the macrophages to release cyanide ions and alter the oxygen tension and the pH in their vicinity (Larsen A, Stoltenberg M. & Danscher, G (2007) In vitro liberation of charged gold atoms. Autometallographic tracing of gold ions released by macrophages grown on metallic gold surfaces 128, 1-6 Histochem Cell Biol.; Ferre N, Claria, J (2006) New insight into the regulation of liver inflammation and oxidative stress. Mini Rev. Med. Chem. 6, 1321-1330). The macrophages release cyanide into the dissolution membrane and the following chemical process occurs:

$$4Au + 8CN^- + 2H_2O + O_2 = 4[Au(CN)_2]^- + 4OH^-$$

Without the wish to be bound by any theory, it is believed that the complex ion aurocyanide $Au(CN)_2^-$, which is a relatively stable ion, prevents or reduces the microorganisms', e.g., bacteria's, ability to form biofilm. It is believed that $Au(CN)_2^-$ easily penetrates the biofilm and disrupts bacterial activity to prevent biofilm formation.

The length in the largest dimension of the gold particles may vary according to the size and form of the gold particles and may be in the range of 20 $\mu$m to 1000 $\mu$m. The effect of using gold particles or gold implants having a length in the largest dimension in the range of 20 -1000 $\mu$m instead of smaller gold particles, such as gold nanoparticles, or gold salts, is that the >20 micron sized gold particles will not migrate from the position where they are applied and thus, they are kept in proximity to the soft tissue filler where the macrophage-liberated gold ions are needed to prevent and/or reduce biofilm. Gold particles smaller than 20 $\mu$m are on the other hand readily phago-cytosed and removed from the tissue location by phagocytes. Hence, gold particles smaller than 20 $\mu$m, e.g. nanoparticles, will substantially not be subjected to dissolucytotic release of gold ions. Gold particles smaller than 20 $\mu$m, e.g. nanoparticles, are thus very likely not effective in reducing or preventing the formation of biofilm in patients who is to receive, are receiving or have received soft tissue filler. Larger gold particles on the other hand, that being, larger than 1000 $\mu$m will not bring about more gold ions because the gold surface relative to the mass of gold is too little. In addition, larger than 1000 $\mu$m particles can create discomfort or pain for the patient and may be difficult to inject. On top of that, the expenses of larger sized gold will be unnecessarily high.

The pure gold particles of the present invention may be of any shape, however, a length in the largest dimension must be larger than 20 $\mu$m. A shape of the gold particle could for instance be a hollow or a solid shape, a bead, a polygon, a rod, a curled up wire, a coil, a flake, a shaving, a sheet, a ring, a discs, or a biconcave disc. For example, as described in US 7,655,261 B2, the gold particle may be hollow with a cross. As the dissolucytosis process will occur on the surface of the gold particles, it is important that a large surface area to volume ratio be used to maximize the amount of bio-released gold ions (Danscher 2002).

In the context of the present disclosure, a gold flake or a gold particle in the form of a flake should be understood as a substantially flat gold particle with a thickness in the range of 0.01 $\mu$m to 30 $\mu$m.

In a preferred embodiment, the gold particle is in the form of a flake.

In a preferred embodiment, the gold particle is in the form of a flake with a length in the largest dimension in the range of 20 to 1000 $\mu$m.

In a preferred embodiment, the gold particle is in the form of a flake with a length in the largest dimension in the range of 50 to 200 $\mu$m.

In a preferred embodiment, the gold particle is in the form of a flake with a thickness in the range of 0.01 $\mu$m to 30 $\mu$m, such as in the range of 0.01 $\mu$m to 10 $\mu$m, such as in the range of 0.01 $\mu$m to 3 $\mu$m.

Preferably, the gold particle of the present invention has a low sedimentation velocity when suspended in a fluid or a soft tissue filler.

In a preferred embodiment, the gold particle of the present invention has a sedimentation velocity of as less than 1000 $\mu$m/s, such as less than 100$\mu$m/s, or less than 10$\mu$m/s, when suspended in a 20mg/ml hyaluronic acid composition with an elastic modulus G' in the range of 100 to 400 Pa, wherein the hyaluronic acid composition consists of crosslinked hyaluronic acid gel particles suspended in an aqueous solution of non-crosslinked hyaluronic acid, and wherein the crosslinked hyaluronic acid has a molecular weight in the range of 600 to 1400 kDa.

In a preferred embodiment, the gold particle of the present invention has a sedimentation velocity of less than 100 $\mu$m/s, when suspended in a 20 mg/ml hyaluronic acid composition with a viscosity $\eta$ in the range of 100 to 600 Pa, wherein the hyaluronic acid composition consists of crosslinked hyaluronic acid gel particles suspended in an aqueous solution of non-crosslinked hyaluronic acid, and wherein the crosslinked hyaluronic acid has a molecular weight in the range of 600 to 1400 kDa.

In the context of the present disclosure, elastic modulus G' is measured in shear stress at 1 Hz as described by Molliard et al. (Molliard et al. (2018) Key rheological properties of hyaluronic acid fillers: from tissue integration to product degradation. Plast Aesthet Res 5:17).

In the context of the present disclosure, viscosity is measured as described by Sundaram et al. (Sundaram et al. (2010) Comparison of the rheological properties of viscosity and elasticity in two categories of soft tissue fillers: calcium hydroxylapatite and hyaluronic acid, Dermatol. Surg., Nov;36 Suppl 3: 1859-65).

In a preferred embodiment, the gold particle of the present disclosure has a sedimentation velocity of less than 300$\mu$m/s, such as less than 200$\mu$m/s, such as less than 100$\mu$m/s, when suspended in water at 25°C.

In the context of the present disclosure, sedimentation velocity should be understood as the average downward velocity of particle(s) in suspension (velocity brought about by gravity). Methods for determining sedimentation velocity are well known in the art, such as Sedimentation Velocity Analytical Ultracentrifugation. The lower the sedimentation velocity, the longer time it takes for a particle to settle. Most particles settle at a local low point of the suspension container (on the wall of the container and/or on top of other particles) but can also settle at higher points on a wall of the container (and/or on top of other particles). In the context of the present disclosure, a particle is no longer in suspension when it has settled and will need to be suspended, by shaking the suspension, to re-establish the suspension. Thus, the gold particle is preferably administered into soft tissue while in suspension. It also follows that a gold particle, which has a low sedimentation velocity when present in viscous fluids or compositions, advantageously increases the window of time in which the gold particle is preferably administered. Furthermore, when more than one gold particle is present in a suspension, a low sedimentation velocity of the particles results in a slower decrease in suspension homogeneity. Greater homogeneity, in turn, is preferable when administering the suspension of more than one gold particles, since it can result in a more homogenous distribution of gold particles in the tissue of the patient, leading to improved control of the local concentration of gold ions in the tissue.

Several particle properties influence sedimentation velocity, such as particle density and shape. Sedimentation velocity can be generally described through the following equation:

$$v = \frac{\Delta\rho d^2 gC}{18\eta X}$$

Whereby $\Delta\rho$ is the difference in density between particle and the suspension fluid; d is the diameter of the particle; X is the shape correction factor; η is the viscosity; g is the gravitational acceleration; C is the Cunningham correction factor. A large surface area to volume ratio will increase the drag on the particle. Thus, shapes that have a large surface area to volume ratio such as flakes, sheets, rings, or discs, will have lower sedimentations velocities compared to a spherical particle of the same weight. Hence, a gold particle with a flake, sheet, ring, or disc shape is preferred.

Without the wish to be bound by theory, it is believed that gold particles must be placed inside tissue which is in proximity of/next to the soft tissue filler and/or embedded in the soft tissue filler of the patient, to be effective.

In one embodiment of the invention the gold particle is injected into soft tissue of a patient, the soft tissue containing soft tissue filler.

In another embodiment the gold particle is injected into soft tissue which is located in proximity to the soft tissue filler in the patient.

In the context of the present invention, soft tissue fillers should be understood as an injectable composition that may be injected into the soft tissue of a patient for reconstructive and/or cosmetic purposes. The composition is typically a water solution and/or suspension (colloid) comprising a natural or synthetic compound, preferably a sugar molecule, a fat molecule, hyaluronic acid, collagen, a polymer including poly(methyl methacrylate) (PMMA), a biopolymer, polylactide, acryl, or a combination thereof.

Soft tissue fillers are generally categorized as temporary, semi-permanent, and permanent fillers. The most common temporary fillers are based on hyaluronic acid and comprises brand names such as Restylane and Juvéderm. The filling effect of temporary fillers usually lasts in the range of 6 to 12 months. The most common semi-permanent fillers are based on calcium hydroxyapatite and comprises brand names such as Radiesse and Sculptra. The filling effect of semi-permanent fillers usually lasts in the range of 1 to 2 years. Permanent fillers are based on polymethylmethacrylate and fat and include brand names such as ArteFill.

In soft tissue fillers comprising hyaluronic acid, the hyaluronic acid is often in two forms: crosslinked and non-crosslinked. The crosslinked hyaluronic acid is most commonly synthesized by consumption of carboxyl groups. Furthermore, the crosslinked form of hyaluronic acid most often forms gel particles, and in soft tissue fillers, the gel particles are most often suspended in a solution of non-crosslinked hyaluronic acid. The solution of non-crosslinked hyaluronic acid acts as a lubricant, allowing the suspension of gel particles to be pushed through a fine needle. The most widely used soft tissue fillers today are a biphasic gel of crosslinked hyaluronic acid gel particles suspended in a solution of non-crosslinked hyaluronic acid. The volume ratio of crosslinked to non-crosslinked hyaluronic acid is commonly approx. 75:25. However, soft tissue fillers comprising hyaluronic acid with no crosslinked hyaluronic acid and soft tissue fillers only consisting of crosslinked hyaluronic acid also exist.

In an embodiment, the soft tissue filler comprises hyaluronic acid.

In an embodiment, the soft tissue filler comprises linear hyaluronic acid.

In an embodiment, the soft tissue filler comprises crosslinked hyaluronic acid.

In an embodiment, the soft tissue filler comprises crosslinked hyaluronic acid and non-crosslinked hyaluronic acid.

In an embodiment, the soft tissue filler comprises crosslinked hyaluronic acid gel particles.

In an embodiment, the soft tissue filler comprises crosslinked hyaluronic acid gel particles and an aqueous solution of non-crosslinked hyaluronic acid.

In an embodiment, the soft tissue filler comprises crosslinked hyaluronic acid gel particles and an aqueous solution of non-crosslinked hyaluronic acid, the volume ratio between gel particles and aqueous solution being in the range of 65:35 to 95:5.

In an embodiment, the soft tissue filler comprises crosslinked hyaluronic acid gel particles with an average molecular weight in the range of 600 to 1400 kDa.

The soft tissue filler is preferably a dermal filler, i.e., a filler suitable for injection in the dermis, subcutis, or submucosal layer of a patient. Often, the soft tissue filler is injected in the subcutis.

In an embodiment, the soft tissue filler is a subcutaneous filler, i.e., a filler suitable for subcutaneous injection.

Soft tissue filler can be injected by several different techniques that depend on the treating physician's experience and preference, and patient characteristics. Usually, treatments involve several injections. The most used procedures are serial puncture, linear threading, and serial threading, and cross-hatching.

In an embodiment, the soft tissue filler is/was administered to the patient by means of multiple needle passes, the needle passes being performed with the same needle.

Soft tissue filler is often injected in on ore more soft tissue areas of the face, such as in a lip, a cheek, the forehead, the chin, and/or the nose. Another common site of injection is the buttocks. Soft tissue filler is also often commonly used for scars, such as acne scars, chicken pox scars, scars from injuries, scars from surgery, and depressed areas of the skin caused by burn scars. Most often, use of filler for scars is to improve the appearance of facial scars. However, dermal fillers are appropriate for use on any part of the body as its use can help manage the appearance of bodily scarring. For example, soft tissue filler is often used for improving the appearance of chicken pox scars on the chest and décolletage, or acne scarring on the back.

In an embodiment, the soft tissue filler is/was administered to the patient by injection into a dermis of a patient, such as the mid-to-deep dermis.

In another embodiment, the soft tissue filler is/was administered to the patient by injection into a subcutaneous layer of the patient.

In another embodiment the soft tissue filler is/was administered to the patient by injection into a submucosal layer of the patient.

In another embodiment the soft tissue filler is/was administered to the patient by injection into a lip.

In another embodiment the soft tissue filler is/was administered to the patient by injection into a cheek.

In another embodiment the soft tissue filler is/was administered to the patient by injection into an area of a scar.

In another aspect, the present invention relates to a composition comprising one or more gold particle(s) having a length in the largest dimension in the range of 20 - 1000 $\mu$m and a physiologically acceptable carrier for use in therapy to prevent or reduce the formation of biofilm in patients who are to receive, are receiving, or have received soft tissue filler.

Hereby, advantages and effects similar to the ones achieved according to other aspects of the invention may be achieved.

In the context of the present disclosure, a physiologically acceptable carrier should be understood as a carrier or excipient that is useful in preparing a pharmaceutical composition that is generally safe non-toxic and neither biologically nor otherwise undesirable.

In an embodiment, the physiologically acceptable carrier is a soft tissue filler.

In another embodiment, the physiologically acceptable carrier comprises hyaluronic acid.

In an embodiment, the one or more gold particle(s) comprise a gold particle that is in the form of a flake or wherein the one or more gold particle(s) is/are (a) gold particle(s) in the form of (a) flake(s).

In an embodiment, the composition according to the invention has a gold mass concentration of at least 0.5 $\mu$g/ml, such as at least 0.1 mg/ml, such as at least 1 mg/ml, at least 5 mg/ml, or at least 10 mg/ml.

In an embodiment, the composition according to the invention has a gold particle concentration of at least 1000 gold particles/ml, such as at least 8000 gold particles/ml, such as at least 20,000 gold particles/ml, or at least 100,000 gold particles/ml.

In another aspect, the present invention relates to a medical device operable to prevent or reduce the formation of biofilm in patients who are to receive, are receiving, or have received soft tissue filler, the medical device comprising: a composition comprising gold particles having a length in the largest dimension in the range 20 - 1000 $\mu$m and a physiologically acceptable carrier.

Hereby, advantages and effects similar to the ones achieved according to other aspects of the invention may be achieved.

In an embodiment, the composition is operable to be injected by means of the medical device within a dermis and/or a submucosal layer of a patient.

In another embodiment, the medical device comprises means for suspending the gold particles under aseptic conditions.

In an embodiment, means for suspending the gold particles under aseptic conditions comprises a first fluid chamber, a second fluid chamber, and a third fluid chamber having a volume smaller than at least one of the first and second fluid chambers, the first and the second fluid chambers being fluidly connected via the third fluid chamber and the third fluid chamber being adapted to be detachable from the first fluid chamber or the second fluid chamber, a first hand-operable piston arranged inside the first fluid chamber and a second hand-operable piston arranged inside the second fluid chamber, the first hand-operable piston contacting an inner wall of the first fluid chambers forming a seal and the second hand-operable piston contacting an inner wall of the second fluid chamber forming a second seal, wherein when the first hand-operable piston is pushed, the volume of the first fluid chamber is reduced and the volume of the second fluid chamber is increased, and when the second hand-operable piston is pushed, the volume of the first chamber is increased and the volume of the second chamber is reduced.

In another aspect, the present invention relates to a method of prevention or reduction of biofilm formation in a patient who is to receive, is receiving, or have received soft tissue filler, the method comprising the step of: administering a gold particle having a length in the largest dimension in the range of 20 - 1000 $\mu$m to the dermis and/or submucosal layer of the patient.

Hereby, advantages and effects similar to the ones achieved according to other aspects of the invention may be achieved.

In an embodiment, the method of prevention or reduction of biofilm formation comprises the step of, during the step of administering the gold particle, administering soft tissue filler.

In an embodiment, the method of prevention or reduction of biofilm formation comprises the step of, prior to the step of administering the gold particle, suspending the gold particle in a composition comprising a physiologically acceptable carrier, and wherein, in the step of administering the gold particle, the gold particle is administered to the patient by administration of the composition.

In an embodiment, the method of prevention or reduction of biofilm formation comprises the step of suspending the gold particles, the suspending being performed under aseptic conditions.

In an embodiment, the method of prevention or reduction of biofilm formation comprises the step of, prior to the step of administering the gold particle, suspending the gold particle in a composition comprising a physiologically acceptable carrier, and wherein the physiologically acceptable carrier is a soft tissue filler and/or hyaluronic acid.

A gold particle may be administered before, during, or after the administration of soft tissue filler. However, the greatest effect can be achieved when administering the gold particle before or during the administration of filler. One dose treatment is sufficient to achieve long-term efficacy since the gold will remain for a lifetime in the tissue of the patient where it has been placed. However, several doses may also be administered.

In the context of the present disclosure, aseptic conditions should be understood as substantially free from contamination caused by harmful bacteria, viruses, or other microorganisms, e.g., surgically sterile or sterilized.

In the context of the present disclosure, aseptically sealed should be understood as a seal through which substantially no harmful bacteria, viruses, or other microorganisms can pass.

In the context of the present disclosure, any mass/volume concentration, such as mg/ml, should be understood as the mass per volume at 25°C.

In the context of the present disclosure, any volume to volume ratio should be understood as a volume to volume ratio at 25°C.

In the context of the present disclosure, crosslinking, i.e., the degree of crosslinking, is the stoichiometric ratio between 1,4-butandioldiglycidyl ether residues that are double-linked and hyaluronic acid disaccharide units. Crosslinking is indicated in percentage (%).

**Examples**

Example 1: micron-sized gold particles remain at implanted site in dogs for 35 months

A study supporting the invention has been conducted with 10 dogs aged between 5.5 months and 7 years and 6 months, and weighing between 8 and 54 kg.

The animals were fully anaesthetized after premedication with Atropin®, Plegicil® and metadon®. Propofol® was injected followed by intubation and inhalation anesthetization with Isofluran®.

Gold particles, oval balls consisting of a 300 mm long and 30 micron thick thread of 99.99% pure gold, were placed in connective tissue surrounding a joint.

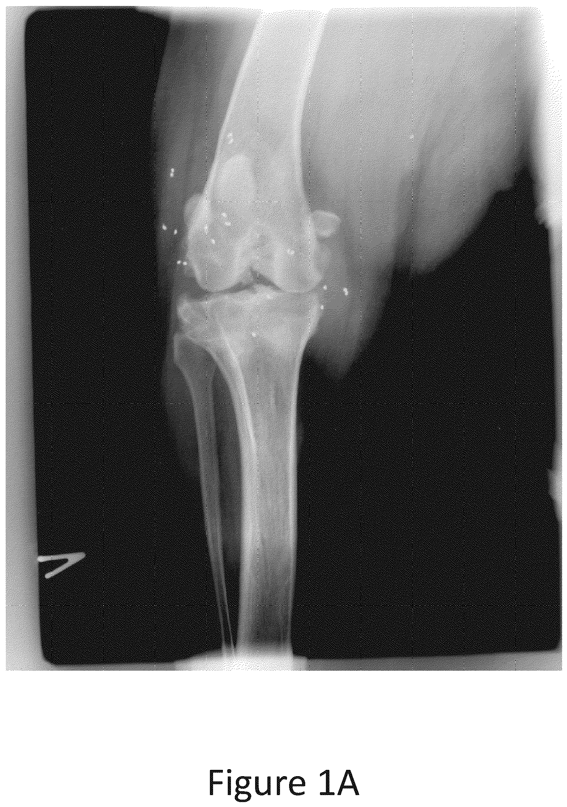

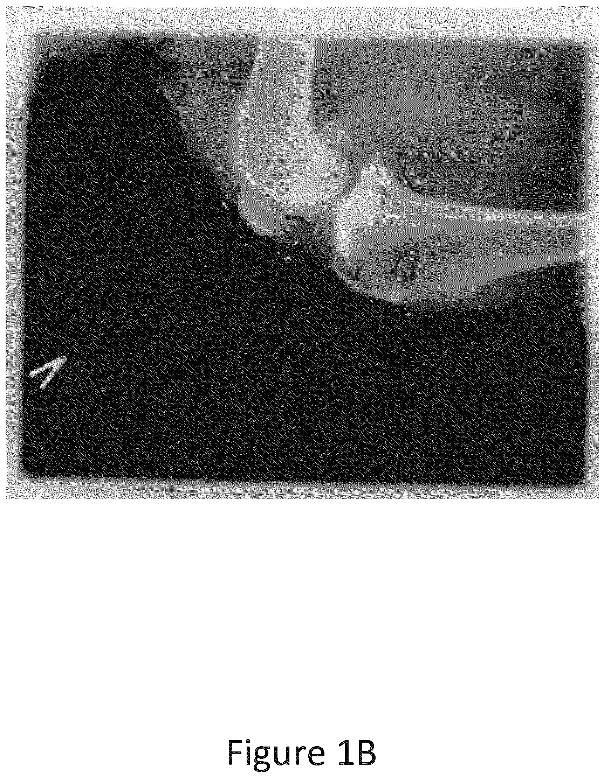

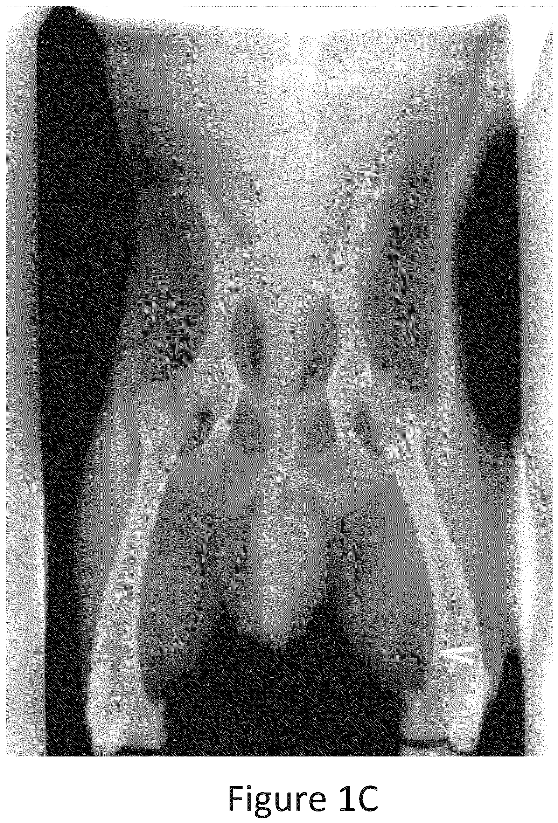

Immediately after implanting the gold particles, X-ray photographs were taken of the gold particle-receiving connective tissue, in order to determine the exact location of the gold particles. In a time span varying from 12 to 35 months after placing the gold particles, the dogs were X-ray-photographed again using the exact same projections as used at the first X-ray, figures 1A-C show such photographs of the same dog.

By comparing the X-ray photographs, any migration of the gold particles would be visible as a difference between the photographs.

Referring again to figures 1A-C showing the X-ray pictures of the dog implanted with a number of gold particles after approximately 1 year, each picture visualizes the position of the implanted gold particles from a different angle. The implanted gold particles are visible as small white dots on each of the pictures. From the pictures it is apparent that the gold particles are still located in the connective tissue around the hip joints where they were implanted about a year earlier.

The results of this study, although the implants were gold particle oval balls consisting of a 300 mm long and 30 micron thick thread of 99,99% pure gold, is thought to be representative of gold particles of any shape, such as gold flakes.

Example 2: Determining the sedimentation velocity of gold particles in soft tissue filler suspensions.

Gold flakes with a length in the largest dimension in the range of 20-1000 were added to a filler consisting of an aqueous solution-suspension of 25.5 mg/ml hyaluronic acid with an elastic modulus G' in the range of 40 to 350 Pa, to a final gold mass concentration of 10 mg/ml. The filler suspension containing gold flakes was then added to a 3.5 ml cuvette and measured by a Horiba Photon Technologies Quantamaster T configuration Fluorescence Spectrometer operated in scattering mode, at 637 nm for 4 hours. No difference in scattering signal was observed during the measurement period, i.e., the gold flakes did not sediment. Based on the size of the cuvette, the gold flake's sedimentation velocity was determined to be <2.7 $\mu$m/s.

**Claims**

1. A gold particle having a length in the largest dimension in the range of 20 - 1000 μm for use in therapy to prevent or reduce formation of biofilm in patients who are to receive, are receiving, or have received soft tissue filler.

2. The gold particle for use in therapy to prevent or reduce the formation of biofilm in patients according to claim 1, wherein the gold particle is in the form of a flake.

3. The gold particle for use in therapy to prevent or reduce the formation of biofilm in patients according to claim 1 or 2, wherein the soft tissue filler comprises hyaluronic acid.

4. A composition comprising one or more gold particle(s), the one or more gold particle(s) having a length in the largest dimension in the range of 20 - 1000 μm and a physiologically acceptable carrier for use in therapy to prevent or reduce the formation of biofilm in patients who are to receive, are receiving, or have received soft tissue filler.

5. The composition for use in therapy to prevent or reduce the formation of biofilm in patients according to claim 4, wherein the physiologically acceptable carrier is a soft tissue filler.

6. The composition for use in therapy to prevent or reduce the formation of biofilm in patients according to claims 4 or 5, wherein the one or more gold particle(s) comprise a gold particle that is in the form of a flake or wherein the one or more gold particle(s) is/are (a) gold particle(s) in the form of (a) flake(s).

7. The composition for use in therapy to prevent or reduce the formation of biofilm in patients according to claims 4-6, wherein the composition has a gold particle concentration of at least 1000 gold particles/ml.

8. The composition for use in therapy to prevent or reduce the formation of biofilm in patients according to claims 4-7, wherein the composition has a gold mass concentration of at least at least 0.5 μg/ml.

9. A medical device operable to prevent or reduce the formation of biofilm in patients who are to receive, are receiving, or have received soft tissue filler, the medical device comprising: a composition comprising a gold particle having a length in the largest dimension in the range 20 - 1000 μm and a physiologically acceptable carrier.

10. The medical device according to claim 9, wherein the composition is operable to be injected within dermis and/or submucosal layer of a patient.

11. The medical device according to claim 9 or 10, wherein the medical device comprises means for suspending the gold particle under aseptic conditions.

12. A method of prevention or reduction of biofilm formation in a patient who is to receive, is receiving, or have received soft tissue filler, the method comprising the step of: administering a gold particle having a length in the largest dimension in the range of 20 - 1000 μm to the dermis and/or submucosal layer of the patient.

13. The method according to claim 12 further comprising the step of, prior to the step of administering the gold particle, suspending the gold particle in a soft tissue filler thereby producing a suspension, and wherein, in the step of administering the gold particle, the gold particle is administered to the patient by administration of the suspension.

14. The method according to claim 12 or 13, wherein the gold particle is in the form of a flake.

15. The method according to any one of the claims 12 to 14, wherein the soft tissue filler comprises hyaluronic acid.

Figure 1A

Figure 1B

Figure 1C

Europäisches
Patentamt

European
Patent Office

Office européen
des brevets

# EUROPEAN SEARCH REPORT

**Application Number**

EP 23 15 3122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/000861 A1 (DANSCHER GORM [DK]) 4 January 2018 (2018-01-04) * page 1, paragraph [0002] * * pages 2, 3, paragraphs [0023], [0028],[0037]-[0039] * * page 4, paragraph [0042] * | 1-15 | INV. A61K33/24 A61P31/00 A61P31/04 |
| Y | AHIWALE S S ET AL: "A Bacteriophage Mediated Gold Nanoparticles Synthesis and Their Anti-biofilm Activity", INDIAN JOURNAL OF MICROBIOLOGY, HISAR, IN, vol. 57, no. 2, 4 February 2017 (2017-02-04), pages 188-194, XP036247093, ISSN: 0046-8991, DOI: 10.1007/S12088-017-0640-X [retrieved on 2017-02-04] * page 190 * | 1-15 | |
| Y | VANARAJ SEKAR ET AL: "Production and Characterization of Bio-AuNPs to Induce Synergistic Effect Against Multidrug Resistant Bacterial Biofilm", JOURNAL OF CLUSTER SCIENCE, SPRINGER US, NEW YORK, vol. 28, no. 1, 29 September 2016 (2016-09-29), pages 227-244, XP036157242, ISSN: 1040-7278, DOI: 10.1007/S10876-016-1081-0 [retrieved on 2016-09-29] * page 236 * * pages 239, 240 * | 1-15 | TECHNICAL FIELDS SEARCHED (IPC) A61K A61P |

-/--

The present search report has been drawn up for all claims

| Place of search | Date of completion of the search | Examiner |
|---|---|---|
| The Hague | 29 June 2023 | Opravz, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 23 15 3122

Europäisches
Patentamt
European
Patent Office
Office européen
des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/194852 A1 (DANSCHER GORM [DK]) 10 July 2014 (2014-07-10) * page 1, paragraph [0009] * * page 2, paragraphs [0012], [0015], [0018] * * pages 7, 8; claims 6, 8-10, 24 * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 29 June 2023 | Opravz, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 15 3122**

This annex lists the patent family members relating to the patent documents cited in the above-mentioned European search report.
The members are as contained in the European Patent Office EDP file on
The European Patent Office is in no way liable for these particulars which are merely given for the purpose of information.

**29-06-2023**

| Patent document cited in search report | | Publication date | Patent family member(s) | | Publication date |
|---|---|---|---|---|---|
| US 2018000861 | A1 | 04-01-2018 | DK | 3263116 T3 | 14-11-2022 |
| | | | EP | 3263116 A1 | 03-01-2018 |
| | | | EP | 4144355 A1 | 08-03-2023 |
| | | | US | 2018000861 A1 | 04-01-2018 |
| | | | US | 2021093662 A1 | 01-04-2021 |
| US 2014194852 | A1 | 10-07-2014 | US | 2014194852 A1 | 10-07-2014 |
| | | | US | 2019022136 A1 | 24-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017156418 A1

- US 7655261 B2

**Non-patent literature cited in the description**

- **LARSEN A ; STOLTENBERG M. ; DANSCHER, G.** In vitro liberation of charged gold atoms. Autometallographic tracing of gold ions released by macrophages grown on metallic gold surfaces. *Histochem Cell Biol.,* 2007, vol. 128, 1-6
- **FERRE N ; CLARIA, J.** New insight into the regulation of liver inflammation and oxidative stress. *Mini Rev. Med. Chem.,* 2006, vol. 6, 1321-1330

- **SUNDARAM et al.** Comparison of the rheological properties of viscosity and elasticity in two categories of soft tissue fillers: calcium hydroxylapatite and hyaluronic acid. *Dermatol. Surg.,* November 2010, vol. 36 (3), 1859-65